# FASCICULE DE BREVET EUROPEEN

(11) **EP 4 291 547 B1**
(45) Date de publication et mention de la délivrance du brevet: **21.08.2024**
(21) Numéro de dépôt: 22707805.2
(22) Date de dépôt: 08.02.2022
(51) Int. Cl.: C07C 67/08, C07C 67/54, C07C 67/60, C07C 69/54

(54) **PROCEDE PERFECTIONNE DE FABRICATION D'ACRYLATES D'ALKYLE DE PURETE ELEVEE**
VERFAHREN ZUR HERSTELLUNG VON HOCHREINEN ALKYLACRYLATEN
IMPROVED PROCESS FOR MANUFACTURING HIGH-PURITY ALKYL ACRYLATES

(30) Priorité: 15.02.2021 FR 2101402
(43) Date de publication de la demande: 20.12.2023
(73) Titulaire: ARKEMA FRANCE, 92700 Colombes (FR)
(72) Inventeur: MORELIERE, Anne, 57501 Saint Avold cedex (FR); TRETJAK, Serge, 57501 Saint Avold cedex (FR); ESCH, Marc, 57501 Saint Avold cedex (FR); HILPERT, Camille, 57501 Saint Avold cedex (FR)
(74) Mandataire: Arkema Patent
(86) Numéro de dépôt international: PCT/FR2022/050228
(87) Numéro de publication internationale: WO 2022/171954

(56) Documents cités:
- WO-A1-2017/153653
- WO-A1-2020/002830

## Description

### DOMAINE TECHNIQUE

La présente invention concerne la fabrication de (méth)acrylates d'alkyle par estérification directe de l'acide (méth)acrylique par l'alcool correspondant.

Elle a pour objet un procédé perfectionné de fabrication de (méth)acrylate d'alkyle en C₄-C₁₀, en particulier d'acrylate de 2-éthylhexyle, comprenant une étape de valorisation des sous-produits lourds générés au cours de cette fabrication, conduisant à une productivité élevée d'un produit répondant aux normes en matière de pureté et d'acidité, dans des conditions énergétiques optimisées.

### ARRIERE-PLAN TECHNIQUE ET PROBLEME TECHNIQUE

L'estérification de l'acide (méth)acrylique est une réaction équilibrée avec génération d'eau qu'il est nécessaire d'éliminer au cours de la réaction pour déplacer l'équilibre dans le sens de la production de l'ester (méth)acrylique.

Les problèmes qui se posent lors de la fabrication des (méth)acrylates d'alkyle en C₄-C₁₀ par estérification directe de l'acide (méth)acrylique, généralement en présence d'une résine cationique comme catalyseur, sont le plus souvent liés à la complexité des étapes de purification nécessaires après l'étape réactionnelle pour obtenir un produit de haute pureté, au détriment en général de la productivité du procédé.

Les composés « lourds » issus de réactions d'addition de Michael se forment spontanément dans les unités de production de monomères (méth)acryliques. Ces réactions parasites sont favorisées à des températures élevées rencontrées en particulier dans les pieds de colonnes à distiller de ces unités.

Ce propos est illustré, à titre d'exemple, avec la synthèse d'acrylate de 2-éthylhexyle. Le procédé industriel consiste à estérifier l'acide acrylique par de 2-éthylhexanol en excès en présence d'une résine échangeuse d'ions. Le mélange réactionnel comprend en fin de réaction de l'acrylate de 2-éthylhexyle, de l'acide acrylique résiduel, le 2-éthylhexanol en excès, les stabilisants classiquement utilisés pour inhiber les réactions de polymérisation, et différentes impuretés résultant de réactions secondaires.

Ainsi l'acide acrylique, le 2-éthylhexanol n'ayant pas encore réagi, l'eau de réaction s'ajoutent à la double liaison de l'acrylate de 2-éthylhexyle pour former principalement :
- l'acryloxy propionate de 2-éthylhexyle (AP2EH) par addition de l'acide acrylique sur l'acrylate de 2-éthylhexyle ;
- l'hydroxy propionate de 2-éthylhexyle (HP2EH) par addition d'eau sur l'acrylate de 2-éthylhexyle ;
- le 2-éthylhexyloxypropionate de 2-éthylhexyle (OPO) par addition du 2-éthylhexanol sur l'acrylate de 2-éthylhexyle.

Une polyaddition ou la formation de composés mixtes est également possible.

D'autres sous-produits lourds formés lors de la synthèse d'acrylate de 2-éthylhexyle sont des oléfines, comme le 2-éthylhex-2 ène et le 3- méthylhetp-2ène ou l'acide 3-hydroxypropanoïque (AHP) par hydrolyse du dimère d'acide acrylique (di-AA) avec l'eau de la réaction.

Une des caractéristiques des adduits de Mickael est que leur point d'ébullition se trouve au-dessus des points d'ébullition de l'acide acrylique (AA), du 2-éthylhexanol et de l'acrylate de 2-éthylhexyle (A2EH). Comme leur volatilité est faible, ils s'accumulent en fond de la dernière colonne de distillation, en pied de l'évaporateur servant à concentrer ce résidu.

Le résidu de l'évaporateur, outre ces sous-produits lourds et quelques pourcents de monomères libres, contient également une forte concentration en inhibiteurs de polymérisation accumulés au fur et à mesure des étapes de purification, comme la phénothiazine sous sa forme libre ou d'adduit de l'AA ou de l'A2EH, ainsi que des composés de nature polymérique plus ou moins solubles dans le milieu. En général ce résidu est souvent éliminé par incinération, ce qui entraîne une perte de rendement importante.

Ces sous-produits posent un problème de pertes de matières premières et un problème de séparation pour être facilement éliminés, d'autant plus qu'ils peuvent former des azéotropes avec l'ester recherché. Il est donc difficile d'atteindre un ester (méth)acrylique de haute pureté. En outre, la plupart des domaines d'application, notamment celui des adhésifs sensibles à la pression (PSA), requièrent la préparation de polymères (méth)acryliques à partir de monomères répondant à des normes strictes de pureté (> 99,7%) et exempts d'acidité, en particulier comportant une teneur en impuretés liées à l'acide (AHP + di-AA ou AA) inférieures à 90 ppm. Pour limiter ces impuretés, le document EP 3 174 844 décrit l'utilisation d'un excès d'alcool et la mise en circulation d'une boucle réactionnelle comprenant le réacteur d'estérification et une colonne de distillation éliminant l'eau produite sous forme d'un azéotrope avec l'alcool estérifiant. Ce procédé conduit à un ester purifié contenant de faibles traces d'impuretés liées à l'acide.

Pour résoudre ce problème, le document WO 2020/234519 décrit la mise en oeuvre d'une colonne de distillation équipée d'un soutirage latéral et placée en sortie de la section réactionnelle ; celle-ci permet de purger en continu de façon latérale une solution concentrée en AHP et dimères d'acide acrylique, et réduit ainsi la quantité d'impuretés acides résiduelles dans le produit purifié. Cependant, ce document ne mentionne pas le devenir des oléfines dans cette mise en oeuvre

WO2017/153653 A1 décrit la préparation d'un (méth)acrylate d'alkyle par estérification de l'acide (méth)acrylique avec un alcool en C1-C10. Les produits lourds sont soumis à un craquage thermique et une fraction des produits ainsi obtenus est recyclée.

Il subsiste donc un besoin d'améliorer l'élimination des impuretés acides, en particulier l'élimination de l'acide 3-hydroxypropanoïque (AHP), ainsi que des oléfines, dans les procédés de synthèse/purification des esters acryliques décrits dans l'art antérieur.

Il a maintenant été découvert que la mise en oeuvre d'un craquage thermique, avec ou sans catalyseur, permet de transformer avec un haut rendement des adduits de Mickael, sans dépôts solides dans l'installation, dans un procédé permettant l'obtention d'un ester en C₄-C₁₀, en particulier de l'acrylate de 2-éthylhexyle, de haute pureté.

La présente invention a donc pour objet un procédé de récupération/purification d'acrylate d'alkyle, simple à mettre en oeuvre, conduisant à un produit répondant aux normes en matière de pureté avec une productivité optimisée, tout en limitant la taille des équipements à mettre en oeuvre et le coût énergétique.

### RESUME DE L'INVENTION

L'invention a pour objet un procédé de récupération/purification d'un ester acrylique en C₄-C₁₀, à partir d'un mélange réactionnel brut obtenu par estérification directe de l'acide acrylique par l'alcool correspondant, dans lequel un flux riche en impuretés acides telles que l'acide β-hydroxypropionique et l'acide β-acryloxypropionique est soutiré par une sortie latérale lors de la distillation du mélange réactionnel brut, caractérisé en ce qu'il comprend une étape de craquage thermique des résidus de pied de la colonne de purification, conduisant à l'obtention de produits de craquage qui sont recyclés dans le procédé.

Le procédé selon l'invention décrit en détail le cas particulier de la synthèse de l'acrylate de 2-éthylhexyle.

Avantageusement, les produits de craquage sont réintroduits hors zone réactionnelle, ce qui permet de supprimer les effets négatifs des oléfines sur les pertes d'acide acrylique et d'éviter la formation sur la résine catalytique d'hydroxypropionate de 2-éthylhexyle (HP2EH) par estérification de l'acide 3-hydroxypropanoïque (AHP) avec le 2-éthylhexanol.

Par « flux riche en impuretés acides », on entend que l'essentiel de ces impuretés acides générées lors de la réaction d'estérification est présent dans le flux qui est soutiré de façon latérale de la colonne de distillation alimentée par le mélange réactionnel brut. Ce flux comprend en outre de l'ester acrylique, des traces de réactifs non réagis et des sous-produits lourds de point d'ébullition supérieur à celui de l'ester acrylique, ainsi que des traces d'eau. Le flux riche en impuretés acides soutiré peut être sous forme gazeuse ou sous forme liquide, de préférence sous forme liquide.

Le soutirage latéral est de préférence effectué à un niveau plus bas que le niveau d'alimentation de la colonne de distillation, ce qui permet de minimiser la présence des réactifs valorisables tels que l'acide acrylique et l'alcool estérifiant, dans le flux soutiré.

Selon un mode de réalisation du procédé selon l'invention, le flux riche en impuretés acides, soutiré latéralement, est soumis à un traitement avec de l'eau, afin de séparer lesdites impuretés acides et recycler le flux traité dans la colonne de distillation.

Selon un mode de réalisation, le flux traité exempt de l'essentiel des impuretés acides est recyclé dans la colonne de distillation.

Le flux traité exempt de l'essentiel des impuretés acides peut être recyclé dans la colonne de distillation à un niveau plus bas ou à un niveau plus haut que le soutirage latéral, de préférence il est recyclé à un niveau plus haut que le soutirage latéral.

De préférence, le flux traité exempt de l'essentiel des impuretés acides est recyclé dans la colonne de distillation à un niveau plus bas que le niveau d'alimentation de la colonne de distillation.

Le procédé selon l'invention est mis en oeuvre à l'aide d'un système de purification comprenant au moins une colonne de distillation équipée d'un soutirage latéral permettant la séparation de l'essentiel des impuretés acides présentes dans le mélange réactionnel brut. De préférence, ladite colonne de distillation est une colonne d'étêtage séparant en tête les composés légers tels que les réactifs non réagis présents dans le milieu réactionnel.

L'invention a également pour objet un procédé de récupération/purification d'un ester acrylique en C₄-C₁₀, à partir d'un mélange réactionnel brut obtenu par estérification directe de l'acide acrylique par l'alcool correspondant, comprenant au moins les étapes suivantes :
i) on soumet le mélange réactionnel à un étêtage dans une colonne de distillation équipée d'un soutirage latéral permettant d'obtenir :
   - en tête un flux composé essentiellement des réactifs non réagis ;
   - en pied un flux comprenant l'ester recherché et des sous-produits lourds;
   - par soutirage latéral, un flux riche en impuretés acides ;
ii) on soumet le flux de pied de la colonne d'étêtage à une colonne de rectification permettant de séparer :
   - en tête l'ester recherché purifié ;
   - en pied un flux contenant des sous-produits lourds, qui est concentré sur un évaporateur à film ou distillé dans une colonne d'équeutage afin de recycler à la colonne d'étêtage les composés légers présents, et d'éliminer un résidu final de sous-produits lourds ;
iii) on soumet le flux de pied de la colonne de rectification à un traitement thermique ou thermique et catalytique effectué dans un réacteur (craqueur) placé en sortie de l'évaporateur, permettant de séparer :
   - en tête, un flux de produits valorisables recyclé séparément ou mélangé au flux de tête de l'évaporateur ;
   - en pied, un résidu envoyé vers une station de traitement.

Le procédé selon l'invention peut comprendre en outre une étape iv) de traitement du flux soutiré latéralement :
iv) on soumet le flux riche en impuretés acides à une étape de lavage avec un flux aqueux permettant d'obtenir après décantation,
- une phase aqueuse comprenant la totalité des impuretés acides qui peut être envoyée à une station de traitement biologique ou en partie utilisée comme flux aqueux de lavage, et
- une phase organique, comprenant l'ester recherché, des sous-produits lourds et des traces d'eau et de réactifs, qui est recyclée au moins en partie dans la colonne d'étêtage.

Le procédé selon l'invention peut comprendre en outre une étape v du traitement du flux de tête du réacteur de traitement thermique ou thermique et catalytique (craqueur) :
v) on soumet le flux de tête de ce craqueur à une étape de lavage avec un flux aqueux permettant d'obtenir après décantation,
- une phase aqueuse comprenant la totalité des impuretés acides qui peut être envoyée à une station de traitement biologique ou en partie utilisée comme flux aqueux de lavage, et
- une phase organique, comprenant l'ester, l'alcool et l'acide recherchés ainsi que des sous-produits lourds et des traces d'eau, qui est recyclée au moins en partie à l'alimentation de la colonne d'étêtage ou à l'étape de lavage du soutirage latéral.

Le procédé selon l'invention permet d'obtenir un ester acrylique en C₄-C₁₀ de pureté supérieure ou égale à 99,7%, voire supérieure à 99,8%, et comportant une teneur en impuretés acides (AHP + dimère AA + AA) inférieure à 90 ppm, et enfin une teneur en eau inférieure à 400 ppm, intégrant un procédé thermique ou thermique et catalytique permettant d'effectuer le craquage des adduits de Mickael en réactifs ( acide acrylique et alcool) et en produit fini, augmentant ainsi la productivité du procédé en limitant la quantité de résidu à éliminer.

L'invention s'applique avantageusement à la production d'acrylate de 2-éthylhexyle ou d'acrylate de 2-octyle, répondant aux normes de pureté exigées pour la production de polymères utilisables par exemple dans le domaine des adhésifs ou de revêtements.

Un autre objet de l'invention est un procédé de production d'un ester acrylique en C₄-C₁₀ exempt d'impuretés acides et d'oléfines, par estérification directe de l'acide acrylique par l'alcool correspondant comprenant le procédé de récupération/purification tel que défini ci-dessus.

Avantageusement, ledit traitement thermique ou thermique et catalytique permet de valoriser les adduits de Mickael dans un procédé permettant l'obtention d'un ester en C₄-C₁₀, en particulier de l'acrylate de 2-éthylhexyle, en associant un réacteur permettant de réaliser le craquage des produits de purge du pied de l'évaporateur placé au bas de la colonne de purification de l'acrylate de 2-éthylhexyle, avec éventuellement un décanteur supplémentaire suivant les différents modes d'invention permettant de recycler les produits de tête issus du craquage.

Ainsi les produits de tête de craqueur pourront être traités suivant différents modes de réalisation de l'invention :
- lavés dans un décanteur avec de l'eau puis mélangés au produit de tête de l'évaporateur
- lavés dans un décanteur avec de l'eau puis introduits dans le décanteur déjà présent au soutirage latéral,
- introduits directement dans le décanteur déjà présent sur le soutirage latéral.

D'autres caractéristiques et avantages de l'invention ressortiront mieux à la lecture de la description détaillée qui suit, en référence aux Figures 1 à 7 annexées qui représentent :
[Fig. 1] : schéma de principe d'un premier procédé de purification avec 2 colonnes décrit dans le document EP 3 174 844.
[Fig. 2] : schéma de principe d'un second procédé de purification avec 2 colonnes décrit dans le document EP 3 174 844 auquel est ajouté un craqueur C.
[Fig. 3] : schéma de principe du procédé selon l'invention, avec un ajout d'un craqueur C et un recyclage du flux de tête de craqueur à l'entrée de la colonne d'étêtage.
[Fig. 4] : schéma de principe d'un mode de réalisation du procédé selon l'invention, avec un recyclage du flux de tête de craqueur au décanteur D placé sur le soutirage latéral de la colonne d'étêtage.
[Fig. 5] : schéma de principe d'un mode de réalisation du procédé selon l'invention, avec un recyclage du flux de tête de craqueur C à un décanteur D1 puis recyclage du produit lavé à l'entrée de la colonne d'étêtage.
[Fig. 6] : schéma de principe d'un mode de réalisation du procédé selon l'invention, avec un recyclage du flux de tête de craqueur C à un décanteur D1 puis recyclage du produit lavé au décanteur D placé sur le soutirage latéral de la colonne d'étêtage.
[Fig. 7] : schéma de principe d'un mode de réalisation du procédé selon l'invention, avec un recyclage du flux de tête de craqueur à un décanteur D1 puis recyclage du produit lavé à l'entrée de la colonne d'étêtage.

### DESCRIPTION DETAILLEE DE L'INVENTION

Selon un premier aspect, l'invention concerne un procédé de récupération/purification d'un ester acrylique en C₄-C₁₀, à partir d'un mélange réactionnel brut obtenu par estérification directe de l'acide acrylique par l'alcool correspondant, dans lequel un flux riche en impuretés acides telles que l'acide β-hydroxypropionique et l'acide β-acryloxypropionique est soutiré par une sortie latérale lors de la distillation du mélange réactionnel brut, caractérisé en ce qu'il comprend une étape de craquage thermique des résidus de pied de la colonne de purification, conduisant à l'obtention de produits de craquage qui sont recyclés dans le procédé.

Avantageusement, lesdits produits de craquage qui sont recyclés dans le procédé sont l'ester, les réactifs (alcool, acides), mais également des oléfines comme le 2-éthylhex-2-ène et le 3-méthylhetp-2-ène et des impuretés acides qu'il faut éliminer.

Dans le procédé selon l'invention, la décomposition des adduits de Michael peut être réalisée suivant un mode continu, semi-continu ou batch. Le mode continu correspond au fonctionnement préféré de ce procédé d'estérification. On peut utiliser un réacteur tubulaire, un réacteur agité avec une double enveloppe ou ayant une boucle de chauffage externe avec une circulation forcée ou une circulation naturelle (de type thermosiphon). Les composés valorisables générés par la réaction de craquage sont recueillis après condensation des vapeurs en tête du réacteur ou en tête d'une colonne à distiller surmontant ce dernier.

La température de réaction et la pression dans le réacteur sont reliées de façon à ce que l'on élimine par évaporation les réactifs tels l'acide acrylique ou le 2-éthylhexanol ou le produit final, tout en maintenant dans le milieu réactionnel les adduits de Mickael tel l'hydroxypropionate de 2-éthylhexyle (HP2EH).

La réaction de décomposition est effectuée dans une gamme de températures de 180 à 280°C et plus spécialement entre 200°C to 250°C. La pression maintenue au-dessus du réacteur est comprise entre 10000 Pascal et la pression atmosphérique plus spécialement entre 30000 et 60000 Pascal.

Dans le procédé selon l'invention, la décomposition des adduits de Mickael peut être réalisée en présence ou en absence de catalyseur acide protique comme l'acide sulfurique, l'acide para-toluène sulfonique (APTS). L'absence de catalyseur est préférée, car cela évite de créer une ligne d'introduction particulière du catalyseur au niveau de ce réacteur de craquage et surtout de compliquer le traitement de valorisation thermique du résidu final du fait de la présence de cet acide fort concentré à l'échelle de quelques pourcents.

Le temps de séjour basé sur le débit d'alimentation (kg/h) rapporté au volume réactionnel (1) varie entre 0,5 to 20 heures, de préférence entre 1 et 7 heures.

Le produit de tête de ce craqueur est constitué d'acrylate de 2-éthylhexyle, des réactifs de départ mais également d'oléfines et d'AHP ce qui nécessitera de procéder à un lavage à l'eau puis à une décantation continue dans le décanteur statique ou centrifuge placé sur le soutirage latéral de la colonne ou en mettant en oeuvre un équipement supplémentaire placé directement en sortie de ce craqueur.

L'invention est basée sur la mise en oeuvre d'une purge d'un flux riche en impuretés acides à l'aide d'un soutirage latéral équipant de préférence une colonne d'étêtage dans un procédé de purification d'un mélange réactionnel brut obtenu par estérification directe de l'acide acrylique par un alcool en C₄-C₁₀ ainsi que d'un réacteur permettant de valoriser les adduits de Mickael formés dans ce procédé d'estérification.

L'alcool estérifiant peut-être un alcool aliphatique primaire ou secondaire, comportant une chaine alkyle linéaire ou ramifiée comportant de 4 à 10 atomes de carbone. Comme exemples d'alcools, on peut citer le butanol, le 2-éthylhexanol, le n-octanol, le 2-octanol, le n-décanol et 2-propylheptanol.

De préférence, l'alcool est le 2-éthylhexanol ou le 2-octanol.

La réaction d'estérification est effectuée généralement dans un réacteur surmonté d'une colonne de distillation permettant d'extraire l'eau générée par la réaction. L'eau de réaction est éliminée au fur et à mesure de sa formation sous forme d'azéotrope avec l'alcool estérifiant afin de déplacer l'équilibre d'estérification.

Les conditions opératoires de la réaction d'estérification ne sont pas critiques, le procédé selon l'invention pouvant s'appliquer au mélange réactionnel quel que soit son procédé d'obtention. Ainsi la réaction peut être effectuée en excès d'acide ou en excès d'alcool, à une température généralement comprise entre 70° et 100°C, de préférence entre 75°C et 95°C.

Le réacteur peut être un réacteur à lit fixe ou un réacteur à lit en suspension. La colonne de distillation, surmontant le réacteur est en général à garnissage et elle est équipée d'un condenseur de tête, un décanteur, permettant de décanter les vapeurs condensées en tête et de séparer une phase organique comprenant de l'alcool et des traces d'ester, qui est recyclée dans la colonne, et une phase aqueuse qui est éliminée. La colonne fonctionne généralement à une pression allant de 6000 à 12000 Pascal.

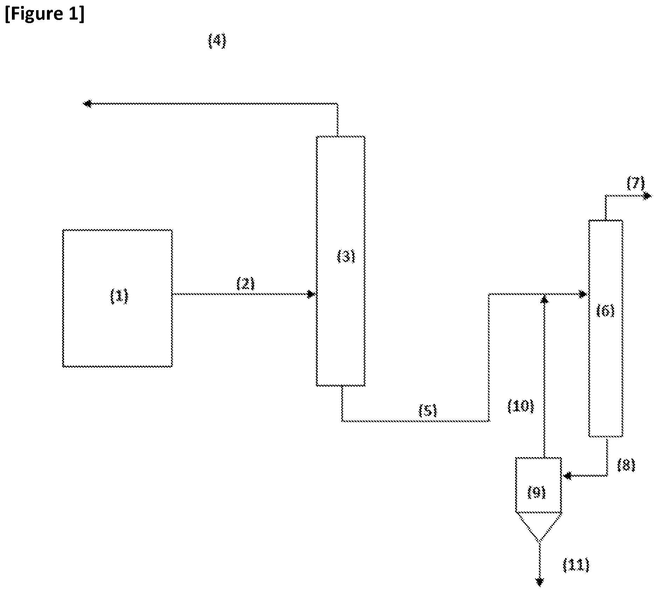

En référence à la Figure 1 qui représente le schéma de principe d'un procédé de récupération/purification d'un ester acrylique selon l'art antérieur décrit dans le document EP 3 174 844, le mélange réactionnel sortant du réacteur alimente la colonne d'étêtage (3) qui sépare essentiellement les réactifs non transformés (4) , de l'ester et des impuretés lourds (5) qui alimentent la colonne de purification (6) qui permet d'obtenir l'ester purifié en tête (7) et un flux (11) résidu de ce procédé après traitement du pied de la colonne (6) sur un évaporateur (9).

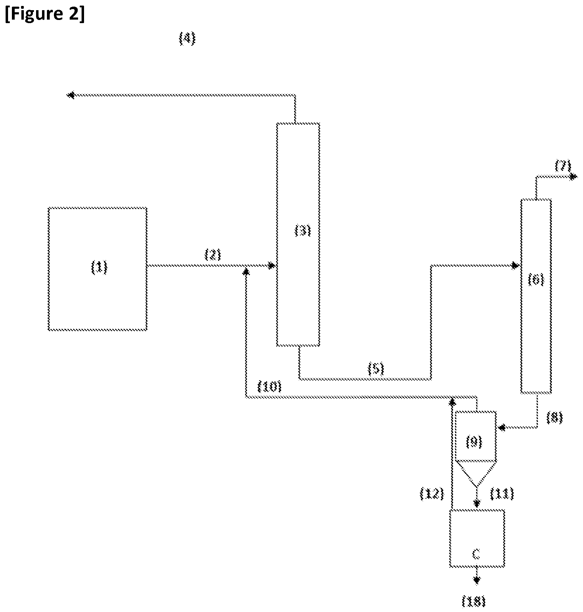

En référence à la Figure 2 qui représente le schéma de principe d'un second procédé, de récupération/purification d'un ester acrylique avec 2 colonnes décrit dans le document EP 3 174 844 auquel est ajouté un craqueur D1. Le mélange réactionnel sortant du réacteur alimente la colonne d'étêtage (3) qui sépare essentiellement les réactifs non transformés (4), de l'ester et des impuretés lourds (5) qui alimentent la colonne de purification (6) qui permet d'obtenir l'ester purifié en tête (7) et un flux (11) résidu de ce procédé après traitement du pied de la colonne (6) sur un évaporateur (9). Enfin le flux (11) alimente un réacteur de craquage C. Le flux de tête de ce dernier est recyclé (12) à l'entrée de la colonne (3).

L'invention remédie aux inconvénients desdits procédés de l'art antérieur, par l'utilisation, en plus d'une colonne de distillation équipée d'un soutirage latéral comme colonne d'étêtage (3), d'un craqueur, conduisant à l'obtention de produits de craquage qui sont réintroduits dans le procédé.

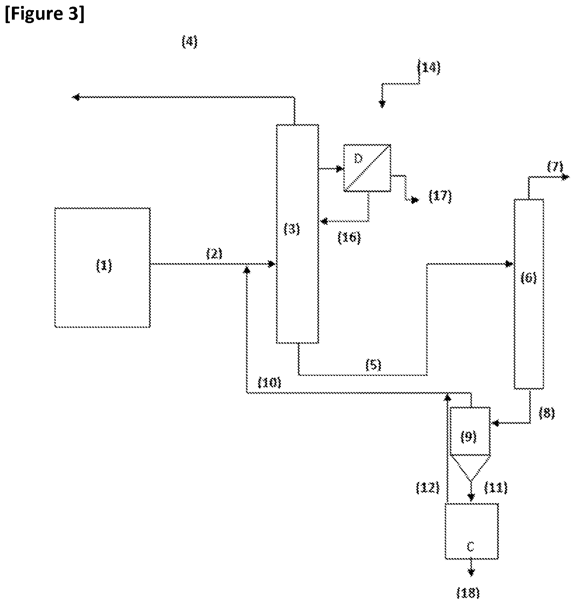

La figure 3 illustre un mode de réalisation du procédé selon l'invention. Le mélange réactionnel sortant du réacteur alimente la colonne d'étêtage (3) qui sépare essentiellement les réactifs non transformés (4), de l'ester et des impuretés lourds (5) ainsi qu'un soutirage latéral permettant de traiter les impuretés acides en lavant ce flux par de l'eau (14), en décantant les phases aqueuses et organiques dans le décanteur D puis en recyclant la phase organique dans la colonne (3). Le pied de la colonne (3) alimente la colonne de purification (6) qui permet d'obtenir l'ester purifié en tête (7) et un flux (11) résidu de ce procédé après traitement du pied de la colonne (6) sur un évaporateur (9). Enfin le flux (11) alimente un réacteur de craquage C. Le flux de tête de ce dernier est recyclé (12) conjointement au flux de tête de l'évaporateur (10) à l'entrée de la colonne (3).

Le résidu 18 de pied de craqueur est envoyé vers une station de traitement.

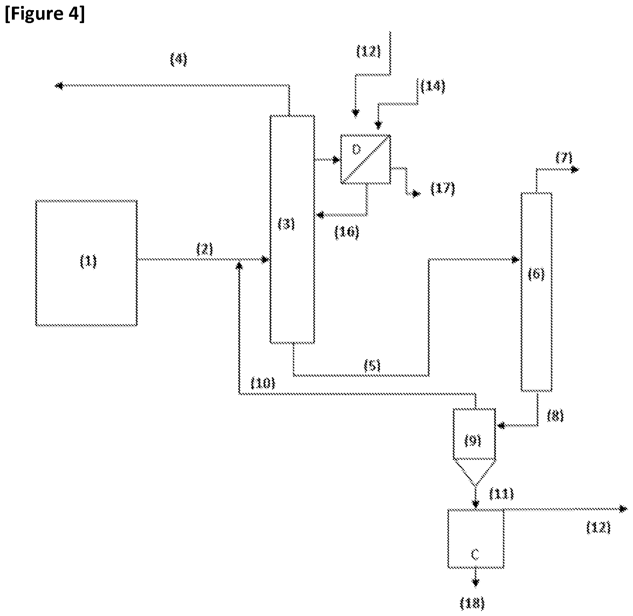

Selon le schéma de principe du procédé selon l'invention, représenté à la Figure 4, un craqueur thermique ou thermique et catalytique (13) est placé en sortie de l'évaporateur 9 permettant de concentrer le pied de la colonne de finition 6. Ce craqueur est alimenté par le flux 11. Le résidu 18 de pied de craqueur est envoyé vers une station de traitement. Le flux de produits valorisables est recyclé au décanteur D placé sur la ligne de soutirage 15 de la colonne 3. Les flux 12 et 15 sont alors lavés avec de l'eau 14. La phase aqueuse est alors envoyée vers une station biologique tandis que le flux organique 16 est recyclé dans la colonne 3.

En référence à la Figure 4, qui représente le mode de réalisation de l'invention préféré, la colonne d'étêtage (3) comporte un équivalent de 10 et 30 plateaux théoriques de préférence 15 à 20 étages théoriques. Les internes utilisés pour la colonne peuvent être des plateaux à clapets ou des plateaux perforés à déversoir, des plateaux à courants croisés comme les Dual Flow les Ripple Trays, les Turbo Grid Shell, ou du garnissage ordonné comme du garnissage structuré telle Mellapack 250X de Sulzer.

L'alimentation de la colonne d'étêtage est constituée du flux provenant de la boucle réactionnelle catalysant la réaction d'estérification de préférence avec une résine cationique forte, par exemple une résine sulfonée de type styrène/divinyle benzène à groupements sulfoniques. A titre d'exemple on peut citer celles commercialisées sous la dénomination LEWATIT K2620 ou K2621 par la société LANXESS ou celles commercialisées sous la dénomination AMBERLYST A15 ; A16 ou A46 par la société ROHM et HAAS.

L'alimentation (2) de la colonne d'étêtage est réalisée au tiers supérieur de cette colonne de préférence entre les plateaux théoriques 3 à 10 comptées à partir de la tête de la colonne.

Le flux (4) de tête de la colonne (3) comprend essentiellement les réactifs non réagis. Ce flux (4) valorisable, est recyclé à la réaction.

La colonne fonctionne avec un taux de reflux (débit de liquide condensé retourné à la colonne/ débit (4) compris entre un ratio de 1/5 à 1/1 de préférence 1/3.

Le flux 15 de soutirage latéral peut être sous forme gazeuse ou liquide de préférence liquide. Le soutirage est placé entre les plateaux théoriques 5 à 15 de préférence entre 8 à 12 comptées à partir de la tête de colonne. L'emplacement de ce soutirage latéral est judicieusement choisi de façon à maximiser la concentration d'AHP et celle de di-AA tout en minimisant la présence de réactifs valorisables (AA et 2-éthylhexanol). Ce soutirage latéral comprend bien entendu la quantité de stabilisants nécessaires à son fonctionnement sans encrassement. On peut si besoin en cas de soutirage en phase gazeuse ajouter également un autre stabilisant. Avantageusement, on introduit de 100 à 5000 ppm d'inhibiteur de polymérisation dans le système de purification selon le procédé de l'invention.

Comme inhibiteurs de polymérisation utilisables, on peut citer par exemple la phénothiazine (PTZ), l'hydroquinone (HQ), l'éther mono méthylique d'hydroquinone (EMHQ), le diterbutyl para-crésol (BHT), la para-phénylène diamine, le TEMPO (2,2,6,6-tétraméthyl-1-piperidinyloxy), le di-tertiobutylcatéchol, ou les dérivés du TEMPO, tel que le OH-TEMPO, seuls ou leurs mélanges en toutes proportions, à des teneurs dans le milieu de réaction pouvant être comprises entre 50 ppm et 5000 ppm, éventuellement en présence d'air appauvri, mais généralement à des teneurs comprises entre 150 ppm et 1000 ppm. L'ajout des inhibiteurs de polymérisation peut se faire à différents endroits, avec l'introduction des réactifs ou en tête de colonne de distillation.

Après refroidissement à la température à laquelle on va faire la décantation de préférence entre 20°C et 70°C, de l'eau (14) est additionnée dans une proportion entre 5 et 100% de préférence entre 30% et 80% par rapport au flux provenant du soutirage latéral (15). Le produit de tête de craqueur (12) refroidi entre 20°C et 70°C est également introduit dans ce décanteur D. Le flux organique lavé (16) est renvoyé dans la colonne entre les plateaux théoriques 5 à 15 de préférence 7 à 12. Ce flux organique contient les oléfines formées qui sont éliminées en tête de la colonne 3. Mélangé, au débit de tête de cette colonne la concentration en oléfines varie peu (3500 ppm à 4000 ppm) et n'entraine de fait pas de problème de fonctionnement du procédé. La phase aqueuse contenant les impuretés liées à l'acide peut être soit recyclé partiellement ou en totalité dans le flux (14) ou évacué vers une station biologique.

Pour rendre les inhibiteurs plus efficaces il convient d'injecter en pied de colonne de l'oxygène, de l'air ou de l'air dit appauvri à 7% O2. De façon préférée la quantité d'oxygène injectée correspond à une teneur de 0,2% à 0,5% rapportée à la quantité de vapeur organique dans la colonne

La colonne peut opérer sous vide, afin de minimiser l'exposition thermique des composés thermosensibles au sein de la colonne. Avantageusement, la colonne (3) fonctionne sous un vide allant de 1333 à 13332 Pascal.

Le flux (5) soutiré en bas de cette colonne et qui va alimenter la colonne d'obtention de l'ester recherché (6) comporte au moins 92% en poids de l'ester recherché, les impuretés liées à l'acide (AHP, di -AA) les impuretés liées à l'alcool et les adduits de Mickael. Ce flux alimente de préférence la colonne (6) entre le plateau théorique 1 à 3 compté à partir du bas de la colonne (6).

La colonne du pur (6) comporte un équivalent de 2 et 15 plateaux théoriques, de préférence 5 à 10 étages théoriques.

La colonne (6) est par exemple une colonne à plateaux perforés ou à garnissage. Les internes utilisés pour la colonne peuvent être des plateaux à clapets ou des plateaux perforés à déversoir, ou des plateaux à courants croisés comme les Dual Flow, les Ripple Trays, les Turbo Grid Shell, ou du garnissage ordonné comme du garnissage structuré telle Mellapack 250X de Sulzer.

Le flux (7) de tête de la colonne (6) est constitué de l'ester recherché ayant comme spécifications une pureté en ester > 99,7%, une teneur en impuretés acide (AHP+diAA +AA) < 90 ppm et enfin une teneur en eau < 400 ppm.

La colonne fonctionne avec un taux de reflux (débit de liquide condensé retourné à la colonne/ débit (7) compris entre un ratio de 1/5 à 1/1, de préférence de 1/5 à 1/2. Comme la colonne (3), celle-ci est stabilisée et de l'air ou de l'air appauvri (7%O2) est injecté en bas de colonne. La colonne peut opérer sous vide, afin de minimiser l'exposition thermique des composés thermosensibles au sein de la colonne. Avantageusement, la colonne (6) fonctionne sous un vide allant de 1333 à 13332 Pa.

Avantageusement la température de fonctionnement est comprise entre 50°C et 160°C.

Le flux de pied (8) est concentré sur un évaporateur à film raclé (9) afin de recycler les composés légers présents vers le début de la section de purification en amont de la colonne (3) ou de la colonne (6) et permet d'éliminer le résidu (11) de produits lourds. Ce résidu alimente un réacteur (13) à recirculation forcée comprenant un échangeur externe chauffé par de la vapeur 33.10⁵ Pascal. La température du milieu réactionnelle est comprise entre 180° et 250°C, de préférence 230°C à 250°C. La pression dans ce réacteur est maintenue entre 13000 Pa et la pression atmosphérique de préférence entre 39000 Pa et 78000Pa au moyen d'une pompe à anneau liquide ou de venturi. Le produit de pied constitue le résidu ultime et il est envoyé vers la filière adéquate. Le produit de tête (12) condensé à une température de 20°C à 30°C est envoyé vers le décanteur D situé sur le soutirage latéral de la colonne (3). Il n'est pas nécessaire d'injecter dans ce réacteur de l'air ou de l'air appauvri car le produit (11) contient tous les stabilisants mis en oeuvre dans le procédé.

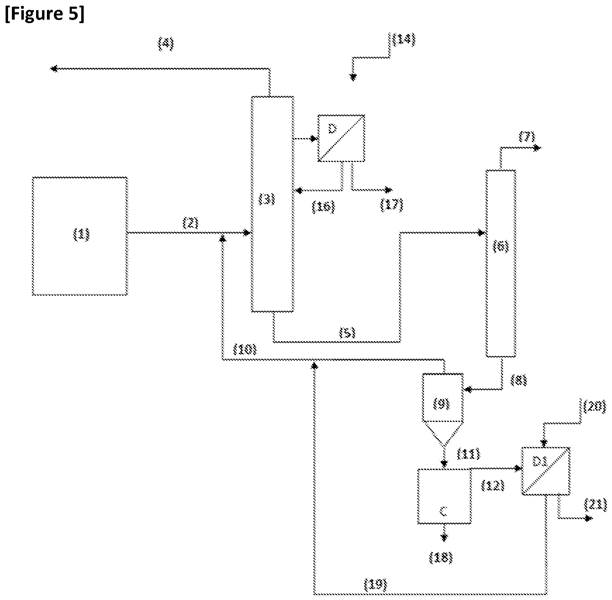

En référence à la Figure 5, est décrit un autre mode de réalisation dans lequel un craqueur thermique ou thermique et catalytique (13) est placé en sortie de l'évaporateur 9, permettant de concentrer le pied de la colonne de finition 6. Ce craqueur est alimenté par le flux 11. Le résidu 18 de pied de craqueur est envoyé vers une station de traitement. Le flux de produits valorisables est recyclé au décanteur D1, lavé avec l'eau 20 puis la phase organique est mélangée au flux 10 et recyclée en entrée de la colonne 3. Le flux aqueux (21) peut être envoyé en station de traitement des eaux ou être recyclé à la réaction pour valoriser l'AA par estérification.

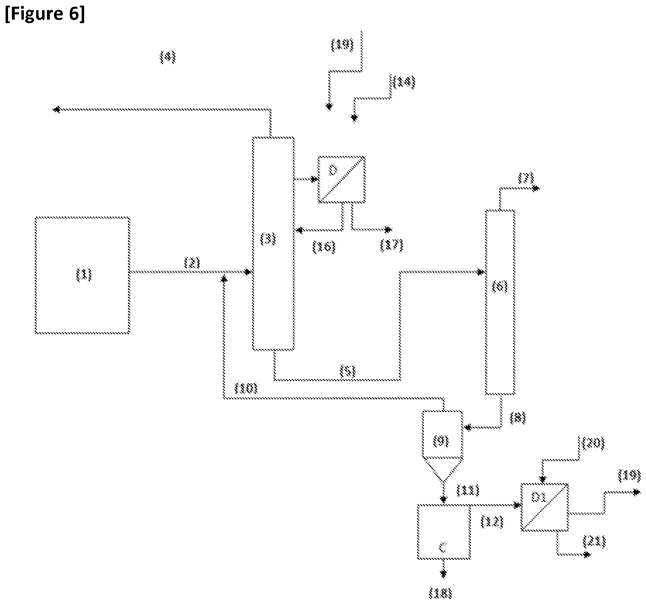

En référence à la Figure 6, est décrit un autre mode de réalisation dans lequel un craqueur thermique ou thermique et catalytique (13) est placé en sortie de l'évaporateur (9), permettant de concentrer le pied de la colonne de finition (6). Ce craqueur est alimenté par le flux 11. Le résidu 18 de pied de craqueur est envoyé vers une station de traitement. Le flux de produits valorisables est recyclé au décanteur D1, lavé avec l'eau (20) puis la phase organique est recyclée au décanteur D dans lequel il est lavé avec l'eau (14) en même temps que le flux de soutirage de la colonne 3. Le flux aqueux (21) peut être envoyé en station de traitement des eaux ou être recyclé à la réaction pour valoriser l'AA par estérification.

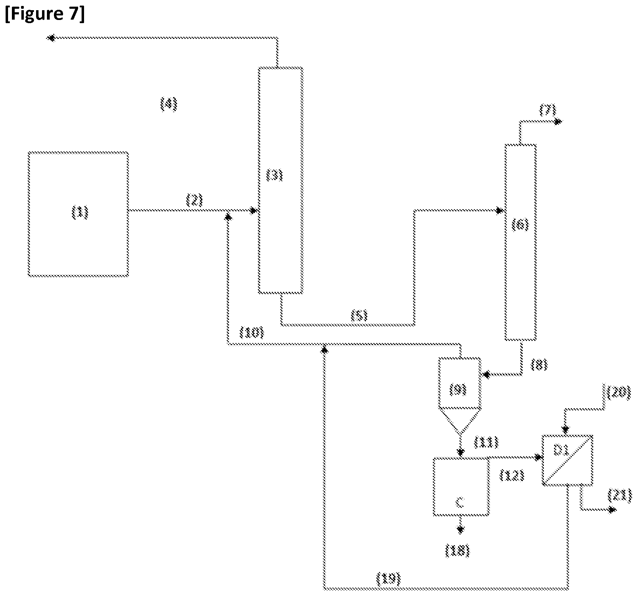

En référence à la Figure 7, est décrit un autre mode de réalisation dans lequel un craqueur thermique ou thermique et catalytique (13) est placé en sortie de l'évaporateur 9 permettant de concentrer le pied de la colonne de finition 6. Ce craqueur est alimenté par le flux 11. Le résidu (18) de pied de craqueur est envoyé vers une station de traitement. Le flux de produits valorisables est recyclé au décanteur D1, lavé avec l'eau (20) puis la phase organique est recyclée à l'entrée de la colonne d'étêtage.

Les exemples ci-après illustrent la présente invention sans toutefois en limiter la portée.

### PARTIE EXPERIMENTALE

Dans les exemples, les pourcentages sont indiqués en poids sauf indication contraire et les abréviations suivantes ont été utilisées :
AA : acide acrylique
AE2H : acrylate de 2-éthylhexyle
E2OH : 2- éthylhexanol
Di-AA : dimères d'AA
HP2EH : Hydroxypropionate de 2-éthylhexyle
AP2EH : Acryloxypropionate de 2-éthylhexyle
OPO : 2-éthylhexyloxypropionate de 2-éthylhexyle
AHP : acide hydroxypropionique
2EHEXENE : 2-ethylhexène
EAU
AIR
Afin de comparer l'art antérieur avec l'invention, les colonnes 3,6 ont une configuration fixée.
Les caractéristiques principales de ces deux colonnes sont les suivantes :
   Colonne 3 : 45 plateaux de type Dual Flow
      Pression tête de colonne : 3333 Pascal
      Alimentation : plateau 15
      Air : plateau 45
      Stabilisant : plateau 1
   Colonne 6 : 26 plateaux de type Dual Flow
      Pression tête de colonne : 2666 Pascal
      Alimentation : plateau 25
      Air : plateau 26
      Stabilisant : plateau 1

### Exemple 1 Procédé A2EH (figure 1)

L'alimentation (2) contient 70 ppm d'AHP.

L'installation dans ce cas de base permet de fabriquer 4950 kg/h soit environ 119 t/j aux spécifications commerciales avec une pureté en AE2H de 99.7% et une teneur en composés acides (di-AA+ AA+3HPA) de 84 ppm.

Les résultats obtenus sont présentés dans le Tableau 1.

**[Tableau 1]**

| Flux | | 2 | 4 | 5 | 7 | 11 |
|---|---|---|---|---|---|---|
| Température | C | 115 | 30 | 143 | 24 | 130 |
| Pression | Pascal | 43000 | 4x10⁵ | 1,5x10⁴ | 3x10⁵ | 4000 |
| Débit massique | kg/hr | 13650 | 7620 | 6300 | 4950 | 440 |

| **Essai 2 fraction massique** | | | | | | |
|---|---|---|---|---|---|---|
| AA | | 0,065 | 0,119 | 0,000 | 0,000 | 0,000 |
| E2HOH | | 0,233 | 0,425 | 0,000 | 0,000 | 0,000 |
| A2EH | | 0,651 | 0,402 | 0,952 | 0,997 | 0,487 |
| EAU | | 0,005 | 0,008 | 0,000 | 0,000 | 0,000 |
| DIAA | ppm | 23,0 | 4,2 | 500,0 | 29,0 | 2565 |
| 2EHEXENE | ppm | 1860,0 | 3380,0 | 0,0 | 0,0 | 0,0 |
| AHP | ppm | 71,0 | 91,0 | 0,0 | 55 | 15 |

### Exemple 2 Procédé A2EH (figure 2)

Un craquer est dans cette exemple mis en oeuvre pour traiter le flux 11. On réinjecte cette fois le flux 12 représentatif de la tête du craqueur (13) avec le flux 10 provenant de la tête de l'évaporateur 9 à l'alimentation de la colonne 3.

La configuration telle qu'envisagée ne permet pas d'obtenir une qualité en composés acides (di-AA+ AA+3HPA) < 90 ppm. Le produit est alors hors spécification avec une teneur en AHP très élevée.

Les résultats obtenus sont présentés dans le Tableau 2.

**[Tableau 2]**

| | | | | | | | | |
|---|---|---|---|---|---|---|---|---|
| Flux | | 2 | 4 | 5 | 7 | 10 | 11 | 12 |
| Température | °C | 115 | 30 | 143 | 24 | 35 | 130 | 20 |
| Pression | Pascal | 43000 | 4x10⁵ | 2x10⁴ | 3x10⁵ | 2x10⁵ | 4000 | 3x10⁵ |
| Débit Massique | kg/hr | 13850,0 | 7620,0 | 6500,0 | 5150,0 | 913,7 | 436,3 | 300,0 |

| % massique | | | | | | | | |
|---|---|---|---|---|---|---|---|---|
| AA | | 0,066 | 0,123 | 0,000 | 0,000 | 0,000 | 0,000 | 0,118 |
| E2HOH | | 0,230 | 0,426 | 0,000 | 0,000 | 0,000 | 0,000 | 0,110 |
| A2EH | | 0,653 | 0,397 | 0,953 | 0,996 | 0,934 | 0,487 | 0,729 |
| EAU | | 0,005 | 0,009 | 0,000 | 0,000 | 0,000 | 0,000 | 0,010 |
| DIAA | ppm | 223,00 | 4,00 | 480,00 | 31,00 | 2032,00 | 2519,00 | 0,00 |
| 2EHEXENE | ppm | 2460,00 | 4529,00 | 0,00 | 0,00 | 0,00 | 0,00 | 30000 |
| AHP | ppm | 135,00 | 145,00 | 124,00 | 145,00 | 41,00 | 40,00 | 2991,00 |

### Exemple 3. Procédé A2EH suivant l'invention (Figure 4)

Le flux 12 de tête de craqueur est recyclé au décanteur D placé sur le soutirage latéral. La quantité d'eau (14) utilisée pour laver le flux 12 et le produit 15 extrait de la colonne ont été fixés à 500 kg/h. L'acidité de l'ester purifié (AHP+ diAA) reste bien inférieure à < 90 ppm. Par ailleurs l'ester produit par le craquage est valorisé comme produit final puisque le débit de production en ester purifié passe de 4950 kg/h à 5150 kg/h) soit une augmentation de la production de 5%.

On peut donc valoriser les adduits de Mickael tout en gardant une pureté très élevée du produit final en augmentant le débit de production.

Les résultats obtenus sont présentés dans le Tableau 3.

**[Tableau 3]**

| | flux | 12 | 5 | 2 | 14 | 17 | 11 | 7 | 4 | 15 | 10 | 16 |
|---|---|---|---|---|---|---|---|---|---|---|---|---|
| Température | C | 20 | 143 | 115 | 20 | 20 | 130 | 24 | 30 | 139 | 35 | 20 |
| Pression | Pascal | 10⁵ | 2x10⁴ | 43000 | 10⁵ | 10⁵ | 4000 | 3x10⁵ | 4x10⁵ | 10000 | 2x10⁵ | 10⁵ |
| Débit massique | kg/hr | 300 | 6500 | 13850 | 500 | 514 | 438 | 5150 | 7905 | 500 | 912 | 786 |

| Fraction Massique | | | | | | | | | | | | |
|---|---|---|---|---|---|---|---|---|---|---|---|---|
| AA | | 0,118 | 0,000 | 0,065 | 0,000 | 0,027 | 0,000 | 0,000 | 0,120 | 0,000 | 0,000 | 0,027 |
| E2HOH | | 0,110 | 0,000 | 0,233 | 0,000 | 0,000 | 0,000 | 0,000 | 0,420 | 0,002 | 0,000 | 0,043 |
| A2EH | | 0,729 | 0,954 | 0,651 | 0,000 | 0,000 | 0,487 | 0,997 | 0,406 | 0,956 | 0,934 | 0,887 |
| EAU | | 0,010 | 0,000 | 0,005 | 1,000 | 0,961 | 0,000 | 0,000 | 0,009 | 0,000 | 0,000 | 0,011 |
| DIAA | ppm | 0,0 | 464,0 | 220,0 | 0,0 | 126,0 | 2436,0 | 30,0 | 4,0 | 167,0 | 1965 | 24,0 |
| 2EHEXENE | ppm | 29910 | 0,0 | 1871,0 | 0,0 | 15,0 | 0,0 | 0,0 | 4440 | 1,3 | 0,0 | 11416 |
| AHP | ppm | 2991,0 | 17,0 | 70,0 | 0,0 | 2810,0 | 5,5 | 20,0 | 43,0 | 1167,0 | 5,8 | 46,0 |

### Exemple 4 : Procédé A2EH suivant l'invention (Figure 6)

Dans cet exemple on met en oeuvre un premier lavage et décantation (D1) sur le flux de la tête du craqueur (12) puis on introduit ce produit au décanteur (D) placé sur le soutirage latéral de la colonne 3.

L'acidité de l'ester purifié (AHP+ diAA) reste bien inférieure à < 90 ppm. Par ailleurs l'ester produit par le craquage est valorisé comme produit final puisque le débit de production en ester purifié passe de 4950 kg/h à 5150 kg/h soit une augmentation de la production de 5%.

Cette solution mettant en oeuvre deux décanteurs fournit les mêmes performances que celles explicitées dans l'exemple 3.

Les résultats obtenus sont présentés dans le Tableau 4.

**[Tableau 4]**

| | flux | **5** | **2** | **8** | **12** | **14** | **20** | **17** | **11** | **7** | **4** | **21** | **15** | **10** | **19** | **16** |
|---|---|---|---|---|---|---|---|---|---|---|---|---|---|---|---|---|
| T | C | 142,9 | 115,0 | 142,8 | 20,0 | 20,0 | 20,0 | 20,0 | 130,0 | 23,8 | 29,7 | 20,0 | 139,0 | 35,0 | 20,0 | 20,0 |
| débit massique fraction massique | kg/hr | 6500 | 13850 | 1350 | 300,0 | 300 | 200 | 304 | 437 | 5150 | 7925 | 189 | 500 | 912 | 311,1 | 495,9 |
| AA | | 0,000 | 0,065 | 0,000 | 0,118 | 0,00 | 0,00 | 0,00 | 0,00 | 0,000 | 0,120 | 0,04 | 0,00 | 0,00 | 0,085 | 0,000 |
| E2HOH | | 0,000 | 0,233 | 0,000 | 0,110 | 0,00 | 0,00 | 0,00 | 0,00 | 0,000 | 0,419 | 0,00 | 0,02 | 0,00 | 0,107 | 0,002 |
| A2EH | | 0,954 | 0,651 | 0,789 | 0,73 | 0,00 | 0,00 | 0,00 | 0,49 | 0,997 | 0,405 | 0,00 | 0,96 | 0,93 | 0,70 | 0,964 |
| EAU | | 0,000 | 0,005 | 0,000 | 0,01 | 1,00 | 1,00 | 0,98 | 0,000 | 0,000 | 0,011 | 0,95 | 0,000 | 0,00 | 0,077 | 0,002 |
| DIAA | ppm | 462 | 220 | 2113 | 2999 | 0 | 0 | 223 | 2431 | 30 | 4 | 0 | 165 | 1 | 2880 | 28 |
| 2EHEXENE | ppm | 0 | 1870 | 0 | 1 | 0 | 0 | 0 | 0 | 0 | 4420 | 77 | 0 | 0 | 28806 | 0 |
| AHP | ppm | 18 | 70 | 6 | 2991 | 0 | 0 | 2047 | 6 | 21 | 46 | 419 | 1254 | 6 | 334 | 9 |

### Exemple 5 : Procédé A2EH suivant la Figure 7

On a évalué dans ce cas l'effet du lavage et de la décantation du flux de tête du craqueur avant injection dans le flux d'alimentation de la colonne 3. Ce décanteur permet d'éliminer les traces supplémentaires d'AHP amenées par le procédé de craquage. En effet la quantité d'AHP présente dans l'ester purifié est la même que celle du procédé de base n'ayant pas mis en oeuvre de craqueur (exemple 2). Toutefois on bénéficie de l'augmentation de productivité de 4950 kg/h à 5150 kg/h liée à l'apport de produit supplémentaire par le craqueur.

Les résultats obtenus sont présentés dans le Tableau 5.

**[Tableau 5]**

| | flux | 5 | 2 | 8 | 12 | 20 | 11 | 7 | 4 | 21 | 10 | 19 |
|---|---|---|---|---|---|---|---|---|---|---|---|---|
| T | C | 143 | 115 | 143 | 20 | 20 | 130 | 24 | 30 | 20 | 35 | 20 |
| Débit Massique Fraction Massique | kg/hr | 6500 | 13850 | 1350 | 300 | 500 | 447 | 5150 | 7919 | 500 | 903 | 300 |
| AA | | 0,000 | 0,066 | 0,000 | 0,118 | 0,000 | 0,000 | 0,000 | 0,119 | 0,032 | 0,000 | 0,065 |
| E2HOH | | 0,000 | 0,233 | 0,000 | 0,110 | 0,000 | 0,000 | 0,000 | 0,420 | 0,000 | 0,000 | 0,110 |
| A2EH | | 0,953 | 0,651 | 0,786 | 0,729 | 0,000 | 0,487 | 0,997 | 0,405 | 0,000 | 0,934 | 0,728 |
| AHP | | 0,000 | 0,000 | 0,000 | 0,000 | 0,000 | 0,000 | 0,000 | 0,000 | 0,000 | 0,000 | 0,000 |
| EAU | | 0,000 | 0,005 | 0,000 | 0,010 | 1,000 | 0,000 | 0,000 | 0,011 | 0,966 | 0,000 | 0,068 |
| DIAA | ppm | 481,0 | 224,0 | 220,0 | 0,0 | 0,0 | 2522,0 | 31,0 | 4,0 | 0,0 | 2035,0 | 0,0 |
| 2EHEXENE | ppm | 0,0 | 1872,0 | 0,0 | 2991,0 | 0,0 | 0,0 | 0,0 | 4420 | 52,0 | 0,0 | 29786,0 |
| AHP | ppm | 47,0 | 71,0 | 15,0 | 2991,0 | 0,0 | 15,0 | 55,0 | 93,0 | 1725,0 | 16,0 | 118,0 |

### Exemple Essais laboratoires 1 : Craquage en continu

Le réacteur à thermosiphon est alimenté en continu à l'aide d'une pompe à membrane.

Le thermosiphon est surmonté d'un condenseur afin de récupéré les vapeurs des produits légers formés.

Une pompe à vide est reliée à la sortie du condenseur à serpentin et également au pot recueillant les lourds ou résidus.

Le flux de lourds est tout d'abord préchauffé jusqu'à 120°C avant d'atteindre le réacteur de façon à maintenir le flux sous sa forme liquide. Le temps de séjour apparent (volume du thermosyphon / débit volumique entrant) est de 4,5 h, la pression est de 50000 Pascal et la température de pied est de 245°C.

L'alimentation initiale contient les composés figurant dans le Tableau 6. Aucun catalyseur n'est ajouté à cette alimentation.

**[Tableau 6]**

| | Alimentation |
|---|---|
| 2-éthylhexanol | 0,9 |
| A2EH | 22,5 |
| HP2EH | 7,8 |
| AP2EH | 20,8 |
| OPO | 16,3 |
| | |
| H2O % | 1,2 |
| AHP (valeur) | 0,2 |
| AA | 0,0 |
| AA2 | 0,6 |
| EMHQ | 0,1 |
| PTZ | 1,4 |
| PTZ/AA | 0,1 |
| PTZ/AE2H | 2,6 |

Le taux d'épuisement (débit de tête / débit d'alimentation) est de 70% en moyenne. Le taux de craquage des adduits principaux est de 96% pour HP2EH, 85% pour AP2EH et 56% pour OPO. La composition du flux de tête est indiquée dans le Tableau 7.

**[Tableau 7]**

| Tête % massique | Tête |
|---|---|
| Somme oléfines en C8 | 0,1-0,2 |
| 2-éthylhexanol | 9,4-10,8 |
| A2EH | 63,1-72 |
| HP2EH | 0,7-1,8 |
| AP2EH | 1,5-3,5 |
| OPO | 0,3-5 |
| AHP | 0,1-0,2 |
| AA | 6,9-10,9 |
| EMHQ | 0,2-0,4 |
| EAU | 1,5-2,4 |

Ce flux contient entre 79 et 94 % de composés valorisables. Il contient également très peu d'oléfines en l'absence de catalyseur. Il contient 0,1 à 0,2 % d'AHP qu'il faudra ensuite éliminer grâce au procédé selon l'invention.

La composition du flux de pied est indiquée dans le Tableau 8.

**[Tableau 8]**

| Résidu % massique | Résidu |
|---|---|
| 2-éthylhexanol | 0,5-0,6 |
| A2EH | 4,4-5 |
| HP2EH | 0,5-0,7 |
| AP2EH | 6,6-7,3 |
| OPO | 23,6-29,6 |
| AA | 0,2 |
| PTZ | 2,9-7,4 |
| PTZ/AE2H | 0,8-1,9 |
| Viscosité Poise | 0,3-0,8 |

Le résidu de pied contient moins de 5% d'A2EH et moins de 40% d'adduits. Les autres composés très lourds (environ 45%) ne peuvent être analysés par chromatographie en phase gazeuse. Sa viscosité est très faible et il ne contient pas de solide qui entraîneraient un bouchage de l'installation.

### Exemple Essais laboratoires 2 : Extraction AHP d'un flux de tête craqueur

Le flux de tête de craqueur est lavé de la façon suivante.

Il est mélangé avec la même masse d'eau à température ambiante.

Le tableau 9 présente la composition de 2 flux de tête de craqueur. Ils contiennent 0,3 et 0,08 % d'AHP et près de 90% de composés valorisables.

**[Tableau 9]**

| % massique | Tête 1 | Tête 2 |
|---|---|---|
| AHP | 0,324 | 0,082 |
| Somme oléfines en C8 | 0,97 | 0,75 |
| 2-éthylhexanol | 12,9 | 13,7 |
| A2EH | 64,9 | 63,2 |
| HP2EH | 0,79 | 0,66 |
| AP2EH | 1,96 | 1,75 |
| OPO | 2,22 | 2,11 |
| AA | 11,9 | 13,6 |
| EAU | 2,1 | 2,1 |

| Composition massique % | Tête 1 lavée | Tête 2 lavée |
|---|---|---|
| AHP final | 0,016 | 0,001 |
| Somme oléfines en C8 | 1,0 | 0,8 |
| 2-éthylhexanol | 13,4 | 14,9 |
| A2EH | 67,8 | 68,6 |
| HP2EH | 0,8 | 0,7 |
| AP2EH | 2,0 | 1,9 |
| OPO | 2,3 | 2,3 |
| AA | 8,3 | 6,8 |
| EAU | 2,4 | 2,1 |

L'AHP est extrait à plus de 94 % et 99% de la phase organique en seulement 1 étage de séparation. Le taux extraction de l'AA est respectivement de 25 et de 55%.

## Revendications

1. Procédé de récupération/purification d'un ester acrylique en C₄-C₁₀, à partir d'un mélange réactionnel brut obtenu par estérification directe de l'acide acrylique par l'alcool correspondant, dans lequel un flux riche en impuretés acides provenant de la réaction d'estérification telles que l'acide β-hydroxypropionique et l'acide β-acryloxypropionique, sont soutirés par une sortie latérale lors de la distillation du mélange réactionnel brut, **caractérisé en ce qu'**il comprend une étape de craquage thermique des résidus de pied de la colonne de purification, conduisant à l'obtention de produits de craquage qui sont recyclés dans le procédé.

2. Procédé selon la revendication 1, dans lequel lesdits produits de craquage recyclés sont des oléfines comme le 2-éthylhex-2-ène et le 3- méthylhetp-2-ène, les impuretés acides, l'ester et les réactifs comme l'alcool ou l'acide.

3. Procédé selon l'une quelconque des revendications 1 et 2, dans lequel le craquage est effectué en l'absence de catalyseur.

4. Procédé selon l'une quelconque des revendications 1 à 3, **caractérisé en ce que** le flux riche en impuretés acides, soutiré latéralement, est soumis à un traitement avec de l'eau, et le flux traité est recyclé dans la colonne de distillation.

5. Procédé de récupération/purification d'un ester acrylique en C₄-C₁₀, à partir d'un mélange réactionnel brut obtenu par estérification directe de l'acide acrylique par l'alcool correspondant, comprenant au moins les étapes suivantes :
i) on soumet le mélange réactionnel à un étêtage dans une colonne de distillation équipée d'un soutirage latéral permettant d'obtenir :
- en tête un flux composé essentiellement des réactifs non réagis ;
- en pied un flux comprenant l'ester recherché et des sous-produits lourds ;
- par soutirage latéral, un flux riche en impuretés acides ;
ii) on soumet le flux de pied de la colonne d'étêtage à une colonne de rectification permettant de séparer :
- en tête l'ester recherché purifié ;
- en pied un flux contenant des sous-produits lourds, qui est concentré sur un évaporateur à film ou distillé dans une colonne d'équeutage afin de recycler à la colonne d'étêtage les composés légers présents, et d'éliminer un résidu final de sous-produits lourds,
iii) on soumet le flux de pied de la colonne de rectification à un traitement thermique ou thermique et catalytique effectué dans un craqueur placé en sortie de l'évaporateur, permettant de séparer :
- en tête, un flux de produits valorisables recyclé séparément ou mélangé au flux de tête de l'évaporateur ;
- en pied, un résidu envoyé vers une station de traitement.

6. Procédé selon revendication 5 comprenant en outre une étape iv) de traitement du flux soutiré latéralement, dans laquelle on soumet le flux riche en impuretés acides à une étape de lavage avec un flux aqueux permettant d'obtenir après décantation,
- une phase aqueuse comprenant la totalité des impuretés acides qui peut être envoyée à une station de traitement biologique ou en partie utilisée comme flux aqueux de lavage, et
- une phase organique, comprenant l'ester recherché, des sous-produits lourds et des traces d'eau et de réactifs, qui est recyclée au moins en partie dans la colonne d'étêtage.

7. Procédé selon la revendication 6 comprenant en outre une étape v) du traitement du flux de tête du craqueur:
- on soumet le flux de tête du craqueur à une étape de lavage avec un flux aqueux permettant d'obtenir après décantation,
- une phase aqueuse comprenant la totalité des impuretés acides qui peut être envoyée à une station de traitement biologique ou en partie utilisée comme flux aqueux de lavage, et
- une phase organique, comprenant l'ester recherché, l'alcool et l'acide réactifs, des sous-produits lourds et des traces d'eau, qui est recyclée au moins en partie à l'alimentation de la colonne d'étêtage ou à l'étape de lavage du soutirage latéral.

8. Procédé selon l'une quelconque des revendications 5 à 7 dans lequel le craqueur est un réacteur tubulaire, un réacteur agité avec une double enveloppe ou ayant une boucle de chauffage externe avec une circulation forcée ou une circulation naturelle.

9. Procédé selon l'une quelconque des revendications 5 à 8, dans lequel la réaction de décomposition est effectuée dans une gamme de températures de 180 à 280°C et plus spécialement entre 200°C to 250°C.

10. Procédé selon l'une quelconque des revendications 5 à 9, dans lequel les produits de tête de craqueur sont lavés dans un décanteur avec de l'eau puis mélangés au produit de tête de l'évaporateur pour être recyclés à l'entrée de la colonne d'étêtage.

11. Procédé selon l'une quelconque des revendications 5 à 9, dans lequel les produits de tête de craqueur sont lavés dans un décanteur avec de l'eau puis introduits dans le décanteur présent au soutirage latéral.

12. Procédé selon l'une quelconque des revendications 5 à 9, dans lequel les produits de tête de craqueur sont introduits directement dans le décanteur présent sur le soutirage latéral.

13. Procédé selon l'une quelconque des revendications précédentes **caractérisé en ce que** l'ester acrylique est l'acrylate de 2-éthylhexyle.

14. Procédé de production d'un ester acrylique en C₄-C₁₀ exempt d'impuretés acides par estérification directe de l'acide acrylique par l'alcool correspondant comprenant le procédé de récupération/purification tel que défini selon l'une quelconque des revendications précédentes.

## Patentansprüche

1. Verfahren zur Gewinnung/Aufreinigung eines C₄-C₁₀-Acrylesters, ausgehend von einer rohen Reaktionsmischung, die durch eine direkte Veresterung von Acrylsäure mit dem entsprechenden Alkohol erhalten wurde, wobei ein Stoffstrom, der reich an sauren Verunreinigungen ist, welche bei der Veresterungsreaktion entstehen, wie etwa β-Hydroxypropionsäure und β-Acryloxypropionsäure, bei der Destillation der rohen Reaktionsmischung über eine seitliche Ausgangsöffnung entnommen wird, **dadurch gekennzeichnet, dass** es einen Schritt des thermischen Crackens der Sumpfrückstände der Aufreinigungskolonne umfasst, wobei dieser bewirkt, dass Crackprodukte erhalten werden, die in das Verfahren zurückgeführt werden.

2. Verfahren nach Anspruch 1, wobei es sich bei den zurückgeführten Crackprodukten um Olefine wie 2-Ethyl-2-hexen und 3-Methyl-2-hepten, um die sauren Verunreinigungen, den Ester und die Edukte wie den Alkohol und den Ester handelt.

3. Verfahren nach einem beliebigen der Ansprüche 1 und 2, wobei das Cracken derart erfolgt, dass keinerlei Katalysator vorliegt.

4. Verfahren nach einem beliebigen der Ansprüche 1 bis 3, **dadurch gekennzeichnet, dass** der an sauren Verunreinigungen reiche Stoffstrom, welcher seitlich entnommen wird, einer Behandlung mit Wasser unterzogen wird und der behandelte Stoffstrom in die Destillationskolonne zurückgeführt wird.

5. Verfahren zur Gewinnung/Aufreinigung eines C₄-C₁₀-Acrylesters, ausgehend von einer rohen Reaktionsmischung, die durch direkte Veresterung von Acrylsäure mit dem entsprechenden Alkohol erhalten wurde, wobei es zumindest die folgenden Schritte umfasst:
i) Entfernen der leichtesten Fraktion aus der Reaktionsmischung in einer Destillationskolonne, die mit einer seitlichen Entnahmeöffnung versehen ist, wobei Folgendes erhalten werden kann:
- kopfseitig ein Stoffstrom, der sich im Wesentlichen aus nicht umgesetzten Edukten zusammensetzt;
- sumpfseitig ein Stoffstrom, welcher den angestrebten Ester und schwerflüchtige Nebenprodukte umfasst,
- durch seitliche Entnahme ein Stoffstrom, der reich an sauren Verunreinigungen ist;
ii) Einwirkenlassen einer Rektifizierungskolonne auf den sumpfseitigen Stoffstrom aus der Kolonne zum Entfernen der leichtesten Fraktion, wodurch Folgendes voneinander getrennt werden kann:
- kopfseitig der aufgereinigte angestrebte Ester;
- sumpfseitig ein Stoffstrom, der schwerflüchtige Nebenprodukte enthält, wobei dieser an einem Dünnschichtverdampfer aufkonzentriert oder einer Destillation in einer Kolonne zum Entfernen der schwersten Fraktion unterzogen wird, um die vorliegenden leichtflüchtigen Produkte in die Kolonne zum Entfernen der leichtesten Fraktion zurückzuführen und einen endgültigen Rückstand schwerflüchtiger Nebenprodukte zu entfernen,
iii) Einwirkenlassen einer Wärme- oder einer Wärme- und Katalysatorbehandlung, die in einer ausgangsseitig des Verdampfers angeordneten Crackanlage erfolgt, auf den sumpfseitigen Stoffstrom aus der Rektifizierungskolonne, wodurch Folgendes voneinander getrennt werden kann:
- kopfseitig ein Stoffstrom von verwertbaren Produkten, der gesondert oder in Mischung mit dem kopfseitigen Stoffstrom des Verdampfers zurückgeführt wird;
- sumpfseitig ein Rückstand, der einer Aufbereitungsanlage zugeführt wird.

6. Verfahren nach Anspruch 5, das darüber hinaus einen Schritt iv) des Behandelns des seitlich entnommenen Stoffstroms umfasst, wobei der Stoffstrom, welcher reich an sauren Verunreinigungen ist, einem Schritt des Waschens mit einem wässrigen Stoffstrom unterzogen wird, wodurch nach dem Absetzenlassen Folgendes erhalten werden kann
- eine wässrige Phase, welche die Gesamtheit der sauren Verunreinigungen umfasst, wobei diese einer biologischen Aufbereitungsanlage zugeführt werden kann oder teilweise als wässriger Stoffstrom zum Waschen verwendet werden kann, und
- eine organische Phase, welche den angestrebten Ester, schwerflüchtige Nebenprodukte sowie Spuren von Wasser und der Edukte umfasst, wobei diese zumindest teilweise in die Kolonne zum Entfernen der leichtesten Fraktion zurückgeführt wird.

7. Verfahren nach Anspruch 6, das darüber hinaus einen Schritt v) des Behandelns des kopfseitigen Stoffstroms der Crackanlage umfasst:
- Einwirkenlassen eines Schritts des Waschens mit einem wässrigen Stoffstrom auf den kopfseitigen Stoffstrom der Crackanlage, wodurch nach dem Absetzenlassen Folgendes erhalten werden kann
- eine wässrige Phase, welche die Gesamtheit der sauren Verunreinigungen umfasst, wobei diese einer biologischen Aufbereitungsanlage zugeführt werden kann oder teilweise als wässriger Stoffstrom zum Waschen verwendet werden kann, und
- eine organische Phase, welche den angestrebten Ester, die Edukte Alkohol und Säure, schwerflüchtige Nebenprodukte und Spuren von Wasser umfasst, wobei diese zumindest teilweise wieder in die Kolonne zum Entfernen der leichtesten Fraktion eingespeist oder wieder dem Schritt des Waschens der seitlich entnommenen Stoffe zugeführt wird.

8. Verfahren nach einem beliebigen der Ansprüche 5 bis 7, wobei es sich bei der Crackanlage um einen Rohrreaktor, einen gerührten Reaktor handelt, der mit einem Doppelmantel oder mit einer externen Heizschleife versehen ist, in welcher eine druckbetriebene Umwälzung oder eine natürliche Umwälzung erfolgt.

9. Verfahren nach einem beliebigen der Ansprüche 5 bis 8, wobei die Zersetzungsreaktion in einem Temperaturbereich von 180 bis 280 °C und insbesondere zwischen 200 °C und 250 °C durchgeführt wird.

10. Verfahren nach einem beliebigen der Ansprüche 5 bis 9, wobei die kopfseitigen Produkte der Crackanlage in einem Absetzbehälter mit Wasser gewaschen und anschließend dem kopfseitigen Produkt des Verdampfers beigemischt werden, um wieder der Eintrittsöffnung der Kolonne zum Entfernen der leichtesten Fraktion zugeführt zu werden.

11. Verfahren nach einem beliebigen der Ansprüche 5 bis 9, wobei die kopfseitigen Crackanlagenprodukte in einem Absetzgefäß mit Wasser gewaschen und anschließend in das Absetzgefäß eingeleitet werden, welches bei den seitlichen entnommenen Stoffen vorliegt.

12. Verfahren nach einem beliebigen der Ansprüche 5 bis 9, wobei die kopfseitigen Crackanlagenprodukte unmittelbar in das Absetzgefäß eingeleitet werden, welches an der seitlichen Entnahmeöffnung vorliegt.

13. Verfahren nach einem beliebigen der vorhergehenden Ansprüche, **dadurch gekennzeichnet, dass** es sich bei dem Acrylester um 2-Ethylhexylacrylat handelt.

14. Verfahren zur Produktion eines C₄-C₁₀-Acrylesters, der frei von sauren Verunreinigungen ist, durch direkte Veresterung von Acrylsäure mit dem entsprechenden Alkohol, wobei es das Gewinnungs-/Aufreinigungsverfahren gemäß der Begriffsbestimmung in einem beliebigen der vorhergehenden Ansprüche umfasst.

## Claims

1. A process for recovering/purifying a C₄-C₁₀ acrylic ester from a crude reaction mixture obtained via the direct esterification of acrylic acid with the corresponding alcohol, in which a stream rich in acidic impurities originating from the esterification reaction, such as β-hydroxypropionic acid and β-acryloxypropionic acid, are withdrawn via a side outlet during distillation of the crude reaction mixture, **characterized in that** it comprises a step of thermal cracking of the residues at the bottom of the purification column, leading to the production of cracking products which are recycled into the process.

2. The process as claimed in claim 1, in which said recycled cracking products are olefins such as 2-ethylhex-2-ene and 3-methylhetp-2-ene, acidic impurities, the ester and the reagents such as the alcohol or the acid.

3. The process as claimed in either of claims 1 and 2, in which cracking is performed in the absence of catalyst.

4. The process as claimed in any one of claims 1 to 3, **characterized in that** the stream rich in acidic impurities, drawn off laterally, is subjected to treatment with water, and the treated stream is recycled into the distillation column.

5. A process for the recovery/purification of a C₄-C₁₀ acrylic ester from a crude reaction mixture obtained by direct esterification of acrylic acid with the corresponding alcohol, comprising at least the following steps:
i) the reaction mixture is subjected to topping in a distillation column equipped with a side draw-off making it possible to obtain:
- at the top, a stream composed essentially of the unreacted reagents;
- at the bottom, a stream comprising the desired ester and heavy byproducts;
- by side draw-off, a stream rich in acidic impurities;
ii) the bottom stream from the topping column is subjected to a rectification column making it possible to separate:
- at the top, the purified desired ester;
- at the bottom, a stream containing heavy byproducts, which is concentrated on a film evaporator or distilled in a tailing column so as to recycle the light compounds present to the topping column, and to eliminate a final residue of heavy byproducts;
iii) the bottom stream of the rectification column is subjected to a thermal or thermal and catalytic treatment performed in a cracker placed at the outlet of the evaporator, making it possible to separate:
- at the top, a stream of upgradable products recycled separately or mixed with the evaporator head stream;
- at the bottom, a residue sent to a treatment plant.

6. The process as claimed in claim 5, also comprising a step iv) of treating the stream drawn off laterally, in which the stream rich in acidic impurities is subjected to a washing step with an aqueous stream, which produces, after decantation:
- an aqueous phase comprising all of the acidic impurities, which can be sent to a biological treatment plant or partly used as aqueous washing stream, and
- an organic phase comprising the desired ester, heavy byproducts and traces of water and of reagents, which is at least partly recycled into the topping column.

7. The process as claimed in claim 6, also comprising a step v) of treating the cracker head stream:
- the cracker head stream is subjected to a step of washing with an aqueous stream, which produces, after decantation:
- an aqueous phase comprising all of the acidic impurities, which can be sent to a biological treatment plant or partly used as aqueous washing stream, and
- an organic phase, comprising the desired ester, the alcohol and acid reagents, heavy byproducts and traces of water, which is at least partly recycled into the topping column feed or into the side draw-off washing step.

8. The process as claimed in any one of claims 5 to 7, in which the cracker is a tubular reactor, a jacketed stirred reactor or a reactor with an external heating loop with forced circulation or natural circulation.

9. The process as claimed in any one of claims 5 to 8, in which the decomposition reaction is performed in a temperature range of from 180 to 280°C and more especially between 200°C to 250°C.

10. The process as claimed in any one of claims 5 to 9, in which the cracker head products are washed in a decanter with water and then mixed with the evaporator head product to be recycled to the inlet of the topping column.

11. The process as claimed in any one of claims 5 to 9, in which the cracker head products are washed in a decanter with water and then introduced into the decanter present at the side draw-off.

12. The process as claimed in any one of claims 5 to 9, in which the cracker head products are introduced directly into the decanter present at the side draw-off.

13. The process as claimed in any one of the preceding claims, **characterized in that** the acrylic ester is 2-ethylhexyl acrylate.

14. A process for producing a C₄-C₁₀ acrylic ester free of acidic impurities by direct esterification of acrylic acid with the corresponding alcohol, comprising the recovery/purification process as defined in any one of the preceding claims.
